(19) Europäisches Patentamt
European Patent Office
Office européen des brevets

(11) **EP 1 890 813 B1**

(12) **EUROPÄISCHE PATENTSCHRIFT**

(45) Veröffentlichungstag und Bekanntmachung des Hinweises auf die Patenterteilung:
**21.03.2018 Patentblatt 2018/12**

(21) Anmeldenummer: **05808201.7**

(22) Anmeldetag: **28.11.2005**

(51) Int Cl.:
*B01J 35/10* (2006.01)    *B01J 21/06* (2006.01)
*C07C 51/31* (2006.01)    *C07C 51/265* (2006.01)
*B01J 35/00* (2006.01)    *B01J 27/198* (2006.01)
*B01J 23/22* (2006.01)    *B01J 35/02* (2006.01)

(86) Internationale Anmeldenummer:
**PCT/EP2005/012701**

(87) Internationale Veröffentlichungsnummer:
**WO 2006/125467 (30.11.2006 Gazette 2006/48)**

(54) **KATALYSATOR SOWIE VERFAHREN ZU DESSEN HERSTELLUNG**

CATALYST AND METHOD FOR PRODUCING IT

CATALYSEUR ET SON PROCÉDÉ DE PRODUCTION

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.05.2005 PCT/EP2005/005546**

(43) Veröffentlichungstag der Anmeldung:
**27.02.2008 Patentblatt 2008/09**

(73) Patentinhaber: **Süd-Chemie IP GmbH & Co. KG 80333 München (DE)**

(72) Erfinder:
• **ESTENFELDER, Marvin
76199 Karlsruhe (DE)**
• **HARTSBERGER, Helmut
81825 München (DE)**
• **GÜCKEL, Christian
85567 Grafing (DE)**

(74) Vertreter: **Graser, Konstanze
Clariant Produkte (Deutschland) GmbH
Patent & License Management
Lenbachplatz 6
80333 München (DE)**

(56) Entgegenhaltungen:
**EP-A- 0 286 448        EP-A- 0 522 871
WO-A-2005/115615      DE-A- 2 106 796
DE-A- 10 040 827      US-A- 3 926 846
US-A1- 2005 076 811**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft einen Katalysator, insbesondere zur Herstellung von Phthalsäureanhydrid (PSA) durch Gasphasenoxidation von o-Xylol und/oder Naphthalin, wobei die katalytisch aktive Masse des Katalysators Titandioxid mit bestimmten Eigenschaften aufweist.

Die großtechnische Produktion von Phthalsäureanhydrid wird durch die katalytische Gasphasenoxidation von o-Xylol und/oder Naphthalin erzielt. Zu diesem Zweck wird ein für die Reaktion geeigneter Katalysator in einen Reaktor, vorzugsweise einen sogenannten Rohrbündelreaktor, indem eine Vielzahl von Rohren parallel angeordnet sind, gefüllt und von oben oder unten mit einem Gemisch aus dem (den) Kohlenwasserstoff(en) und einem sauerstoffhaltigen Gas, beispielsweise Luft, durchströmt. Aufgrund der starken Wärmebildung solcher Oxidationsreaktionen ist es nötig, die Reaktionsröhre zur Vermeidung von sogenannten Hot Spots ("Heißen Flecken") mit einem Wärmeträgermedium zu umspülen und somit die entstandene Wärmemenge abzuführen. Diese Energie kann zur Produktion von Dampf genutzt werden. Als Wärmeträgermedium dient in der Regel eine Salzschmelze und hier vorzugsweise ein eutektisches Gemisch aus $NaNO_2$ und $KNO_3$.

EP 0522871 A1 offenbart einen Katalysator zur Herstellung von Phthalsäureanhydrid durch die katalytische Gasphasenoxidation von ortho-Xylol und/oder Naphthalin mit molekularen Sauerstoff oder einem molekularen Sauerstoff enthaltenden Gas und ein Verfahren zur Herstellung von Phthalsäureanhydrid, das diesen Katalysator verwendet. Der Katalysator wird durch das Aufbringen einer katalytisch aktiven Masse, umfassend Vanadiumoxid; Titandioxid in der Anatas-Modifikation, mit der spezifischen Oberfläche im Bereich von 10 bis 60 $m^2$/g; Niob und mindestens einem Element ausgewählt aus Kalium, Cäsium, Rubidium und Kalium; Phosphor und Antimon auf einen hitzebeständigen anorganischen Träger, hergestellt. Die katalytisch aktive Masse wird durch die Verwendung einer fünfwertigen Antimon-Verbindung als Antimon-Quelle hergestellt.

US 2005/0076811 A1 offenbart ein Verfahren zur Herstellung von Titandioxid mit ultrafeinen Partikeln der Anatas-Modifikation, das im Vergleich zu üblichem Titandioxid, das eine spezifische BET-Oberfläche aufweist, die vergleichbar ist, zu dem des Titandioxids mit ultrafeinen Partikeln, einen niedrigeren Chlorgehalt aufweist. Das Titandioxid mit den ultrafeinen Partikeln hat, gemessen durch Laserbeugung-Partikelgrößenanalyse, einen $D_{90}$-Wert von 2,5 $\mu$m oder weniger.

Ebenso kann man zur Unterdrückung der ungewollten Hot Spots einen strukturierten Katalysator in das Reaktionsrohr füllen, wodurch sich beispielsweise zwei oder drei Katalysatorlagen aus unterschiedlich zusammengesetzten Katalysatoren ergeben können. Solche Systeme sind als solche bereits aus der EP 1 082 317 B1 oder der EP 1 084 115 B1 bekannt.

Die schichtweise Anordnung der Katalysatoren hat auch den Zweck, den Gehalt an unerwünschten Nebenprodukten, d.h. Verbindungen, die in einem möglichen Reaktionsmechanismus von o-Xylol und/oder Naphthalin zu Phthalsäureanhydrid vor dem eigentlichen Wertprodukt stehen, im Roh-PSA so gering wie möglich zu halten. Zu diesen unerwünschten Nebenprodukten zählen hauptsächlich die Verbindungen o-Tolylaldehyd und Phthalid. Die Weiteroxidation dieser Verbindungen zu Phthalsäureanhydrid steigert zudem die Selektivität bzgl. des eigentlichen Wertprodukts.

Neben den oben angesprochenen Unteroxidationsprodukten treten bei der Reaktion auch Überoxidationsprodukte auf. Dazu gehören Maleinsäureanhydrid, Citraconsäureanhydrid, Benzoesäure und die Kohlenoxide. Eine gezielte Unterdrückung der Bildung dieser ungewünschten Nebenprodukte zu Gunsten des Wertprodukts führt zu einem weiteren Anstieg der Produktivität und Wirtschaftlichkeit des Katalysators.

Es besteht ein ständiger Bedarf an Katalysatoren, die einen Anstieg der Produktivität und Wirtschaftlichkeit ermöglichen.

Eine Aufgabe der vorliegenden Erfindung war es daher, einen Katalysator bzw. ein Katalysatorsystem zu entwickeln, das die Nachteile bekannter Katalysatoren aus dem Stand der Technik vermeidet und eine Verbesserung der Aktivität, Selektivität und/oder Lebensdauer des Katalysators ermöglicht.

Nach einem ersten Aspekt betrifft die Erfindung daher einen Katalysator, insbesondere zur Herstellung von Phthalsäureanhydrid durch Gasphasenoxidation von o-Xylol und/oder Naphthalin, mit einem inerten Träger und mindestens einer darauf aufgebrachten Schicht mit einer katalytisch aktiven Masse enthaltend $TiO_2$, dadurch gekennzeichnet, dass zumindest ein Teil des eingesetzten $TiO_2$ folgende Eigenschaften aufweist: (a) die BET-Oberfläche beträgt mehr als 15 $m^2$/g, (b) die Primärkristallitgröße. bestimmt mittels Pulver-Röntgendiffraktometrie nach der Debye-Scherrer-Formel, liegt vorzugsweise bei mehr als 210 Angström wobei der $D_{90}$-Wert des eingesetzten $TiO_2$ zwischen 0,5 und 20 $\mu$m liegt, der $D_{50}$-Wert bei 1,5 $\mu$m oder darunter liegt und weniger als 22% des gesamten Porenvolumens des eingesetzten $TiO_2$ durch Poren mit einem Radius von mehr als 400 nm gebildet werden. Bevorzugte Ausführungsformen sind in den Unteransprüchen angegeben.

Es wird angenommen, ohne dass die Erfindung auf die Richtigkeit dieser theoretischen Annahme beschränkt wäre, dass sich durch die Verwendung des Titandioxids mit den hier beschriebenen Eigenschaften in einem Katalysator besonders vorteilhafte Reaktionsräume für die gewünschten Umsetzungen, insbesondere im Porengefüge, erzielen lassen. Gleichzeitig werden bei Verwendung des erfindungsgemäßen Titandioxids vorteilhafte Zuführwege für die Edukte zu den reaktiven Zentren auf der Oberfläche des Titandioxids sowie Ableitwege für die Reaktions- Produkte bereitgestellt.

Ein wesentliches Merkmal der vorliegenden Erfindung ist die Verwendung des spezifischen Titandioxids, das sich durch eine verhältnismäßig hohe BET-Oberfläche von mehr als 15 $m^2/g$, insbesondere zwischen etwa 15 und 60 $m^2/g$, auszeichnet.

Nach einem weiteren Aspekt der vorliegenden Erfindung wird $TiO_2$ verwendet, das eine Primärkristallitgröße (Primärpartikelgröße)mit mehr als 210 Angström und unter 900 Angström aufweist. Bevorzugterweise weist das Titandioxid einem Primärkristallitgröße von mehr als etwa 210 Angström, insbesondere mehr als etwa 250 Angström, vorzugsweise mehr als etwa 300 Angström, weiter bevorzugt mindestens 320 Angström, insbesondere mindestens etwa 340 Angström, weiter bevorzugt mindestens etwa 380 Angström auf. So wurde gefunden, dass solche $TiO_2$-Primärkristallite mit der vorstehenden (Mindest-)Größe die Herstellung von besonders vorteilhaften Katalysatoren ermöglichen. Bevorzugt liegt die Primärkristallitgröße unter 600 Angström, weiter bevorzugt unter 500 Angström. Die vorstehende Primärkristallitgröße ermöglicht offenbar, ohne dass die Erfindung auf diese Annahme beschränkt wäre, die Ausbildung einer nicht zu kompakten, sondern offenporigen Struktur des Titandioxids im Katalysator. Die Primärkristallitgröße des Titandioxids wird mittels Pulver-Röntgendiffraktometrie nach der Debye-Scherrer-Formel bestimmt, wie im nachstehenden Methodenteil angegeben.

Dabei wurde nach einem bevorzugten Aspekt der vorliegenden Erfindung überraschend gefunden, dass sich bei der Verwendung eines Titandioxids, worin mindestens 25%, insbesondere mindestens etwa 40%, besonders bevorzugt mindestens etwa 50%, am meisten bevorzugt mindestens etwa 60% des gesamten Porenvolumens durch Poren mit einem Radius zwischen 60 und 400 nm gebildet werden, besonders vorteilhafte Katalysatoren erzeugen lassen.

Nach einem weiteren bevorzugten Aspekt der vorliegenden Erfindung wird $TiO_2$ verwendet, dass eine Schüttdichte von weniger als 1,0 g/ml, insbesondere weniger als 0,8 g/ml, besonders bevorzugt weniger als etwa 0,6 g/ml aufweist. Am meisten bevorzugt sind $TiO_2$-Materialien mit einer Schüttdichte von nicht mehr als etwa 0,55 g/ml. Ein Verfahren zur Bestimmung der Schüttdichte ist im nachstehenden Methodenteil angegeben. Es wurde somit gefunden, dass die Verwendung eines Titandioxids mit einer Schüttdichte wie vorstehend definiert, die Herstellung besonders leistungsfähiger Katalysatoren ermöglicht. Es wird angenommen, ohne dass die Erfindung hierauf beschränkt wäre, dass die Schüttdichte hier ein Maß für eine besonders günstige Struktur der im Katalysator zur Verfügung gestellten $TiO_2$-Oberfläche ist, wobei durch die lockere, nicht zu kompakte Struktur besonders günstige Reaktionsräume sowie Zuführ- und Ableitwege für die Reaktanden bzw. Reaktionsprodukte bereitgestellt werden. Nach einer besonders bevorzugten erfindungsgemäßen Ausführungsform wird das verwendete Titandioxid somit neben der hierin beschriebenen Porenradienverteilung und der Primärkristallitgröße auch die hierin definierte Schüttdichte aufweisen. Nach einem weiteren Aspekt der Erfindung wurde jedoch auch gefunden, dass bereits ein Material, dass unabhängig von der hierin beschriebenen Porenradienverteilung und/oder der Primärkristallitgröße die vorstehenden definierte Schüttdichte einhält, unerwartet bessere Ergebnisse aufweist als Vergleichsmaterialien mit einer höheren Schüttdichte.

Nach noch einem weiteren Aspekt der vorliegenden Erfindung sind die Primärkristallite des verwendeten Titandioxids zumindest teilweise zu Agglomeraten verbunden, die z.B. in elektronenmikroskopischen Aufnahmen leicht erkennbar sind. Soweit es sich dabei um offenporige, insbesondere "schwammartige", Agglomerate handelt, wird die bevorzugte nicht zu kompakte, poröse Struktur des Titandioxids begünstigt. Nach einer bevorzugten erfindungsgemäßen Ausführungsform sind die Primärkristallite des $TiO_2$ zu mehr als 30%, insbesondere mehr als 50%, zu Agglomeraten, insbesondere offenporigen Agglomeraten zusammengeschlossen.

Vorzugsweise weist das verwendete $TiO_2$ (Anatas-Modifikation) (in allen Katalysatorlagen) einen Gehalt an Alkali, insbesondere an Na von weniger als 0,3 Gew.-%, insbesondere weniger als 0,2 Gew.-%, bevorzugt weniger als 0,15 Gew.-%, weiter bevorzugt weniger als 0,02 Gew.-%, weiter bevorzugt weniger als 0,015 Gew.-% auf. Vorzugsweise gelten die vorstehenden Grenzwerte für Na und K. Nach einem weiteren bevorzugten Aspekt der Erfindung ist der Anteil an Alkaliverunreinigungen (Gesamtalkaligehalt) des eingesetzten $TiO_2$, bestimmt als die Summe der Lithium-, Natrium-, Kalium-, Rubidium- und Cäsiumverunreinigungen, kleiner als 1000 ppm, insbesondere kleiner 500 ppm, besonders bevorzugt kleiner 300 ppm. Ein Verfahren zur Bestimmung des Anteils an Alkaliverunreinigungen des eingesetzten $TiO_2$ ist nachstehend vor den Beispielen angegeben (DIN ISO 9964-3). Der vorstehende Gesamtalkaligehalt des $TiO_2$ ermöglicht eine exakte Einstellung des Alkali-Promotorgehaltes des Katalysators.

Der Anteil an Alkaliverunreinigungen kann, wie dem Fachmann geläufig, im Bedarfsfall durch Waschen z.B. mit verdünnter Salpetersäure bei erhöhter Temperatur gesenkt werden, um den bevorzugten Bereich von kleiner 1000 ppm zu erreichen. Z.B. kann das $TiO_2$ in 0,1 M $HNO_3$ aufgeschlämmt und unter Rückfluß und unter Agitation bei 90°C über Nacht gewaschen, anschliessend filtriert und dreifach mit bidestilliertem Wasser gewaschen und bei 150°C an Luft getrocknet werden. Anschließend wird der Anteil an Alkaliverunreinigungen erneut bestimmt, und, soweit zu hoch, die vorstehende Prozedur wiederholt.

Nach einer besonders bevorzugten Ausführungsform der vorliegenden Erfindung wird der $TiO_2$-haltige Katalysator zur Gasphasenoxidation von Kohlenwasserstoffen eingesetzt. Insbesondere bevorzugt ist Verwendung zur Herstellung von Phthalsäureanhydrid durch Gasphasenoxidation von o-Xylol, Naphthalin oder Gemischen davon. Es sind jedoch auch eine Vielzahl von anderen katalytischen Gasphasenoxidationen von aromatischen Kohlenwasserstoffen wie Benzol, Xylolen, Naphthalin, Toluol oder Durol zur Herstellung von Carbonsäuren und/oder Carbonsäure- anhydriden im Stand der Technik bekannt. Dabei werden beispielsweise Benzoesäure, Maleinsäureanhydrid, Phthalsäureanhydrid, Isophthalsäure, Terephthalsäure oder Pyromellithsäureanhydrid gewonnen. Auch bei solchen Umsetzungen kann der erfindungsgemäße Katalysator eingesetzt werden.

Im Allgemeinen wird bei der Umsetzung ein Gemisch aus einem molekularen Sauerstoff enthaltenden Gas, beispielsweise Luft, und dem zu oxidierenden Ausgangsmaterial durch einen Festbettreaktor, insbesondere einen Rohrbündelreaktor, der aus einer Vielzahl parallel angeordneter Rohre bestehen kann, geleitet. In den Reaktorrohren befindet sich jeweils eine Schüttung aus mindestens einem Katalysator. Auf die Vorzüge einer Schüttung aus mehreren (unterschiedlichen) Katalysatorlagen wurde oben bereits eingegangen.

Beim Einsatz der erfindungsgemäßen Katalysatoren zur Herstellung von Phthalsäureanhydrid durch Gasphasenoxidation von o-Xylol und/oder Naphthalin wurde überraschend festgestellt, dass die erfindungsgemäßen Katalysatoren eine hohe Aktivität bei gleichzeitig geringer Bildung der unerwünschten Nebenprodukte $CO_x$, d.h. $CO_2$ und $CO$, erhalten wird. Desweiteren zeigt sich eine sehr gute $C_8$- und PSA-Selektivität , wodurch in Summe die Produktivität des Katalysators erhöht wird. In vielen Fällen wird insbesondere auch die hohe $C_8$-Selektivität und die geringe $CO_x$-Selektivität der erfindungsgemäßen Katalysatoren von Interesse sein. Durch die geringe $CO_x$-Selektivität ergibt sich auch in vorteilhafter Weise eine geringere Wärmeentwicklung sowie niedrigere Hot Spot-Temperaturen. Dadurch kommt es zu einer langsameren Deaktivierung des Katalysators im Hot Spot-Bereich.

Nach einer bevorzugten erfindungsgemäßen Ausführungsform weist das eingesetzte $TiO_2$ eine BET-Oberfläche (DIN 66131) zwischen etwa 15 und 45 $m^2/g$, insbesondere zwischen etwa 15 und 30 $m^2/g$ auf.

Weiterhin wird bevorzugt, dass bis zu 80%, insbesondere bis zu 75%, besonders bevorzugt bis zu 70%, des gesamten Porenvolumens durch Poren mit einem Radius zwischen 60 und 400 nm gebildet werden.

Die Bestimmung der hierin angegebenen Porenvolumina bzw. -anteile erfolgt, soweit nicht anders angegeben, mittels Quecksilberporosimetrie (gemäß DIN 66133). Die Angabe des Gesamtporenvolumens bezieht sich dabei in der vorliegenden Beschreibung jeweils auf das gesamte mittels Quecksilberporosimetrie gemessene Porenvolumen zwischen 7500 und 3,7 nm Porenradiengröße.

Poren mit einem Radius von mehr als 400 nm stellen erfindungsgemäß weniger als 22%, bevorzugt weniger als 20%, des gesamten Porenvolumens des eingesetzten $TiO_2$ dar.

Weiterhin bevorzugt wird, dass etwa 50 bis 75%, insbesondere etwa 50 bis 70%, besonders bevorzugt etwa 50 bis 65% des gesamten Porenvolumens des $TiO_2$ durch Poren mit einem Radius von 60 bis 400 nm, und vorzugsweise etwa 15 bis 25% des gesamten Porenvolumens durch Poren mit einem Radius von mehr als 400 nm gebildet werden. Bezüglich der kleineren Porenradien wird bevorzugt, dass weniger als 30%, insbesondere weniger als 20% des gesamten Porenvolumens des Titandioxids, durch Poren mit einem Radius von 3,7 bis 60 nm gebildet werden. Ein hier besonders bevorzugter Bereich beträgt für diese Porengröße etwa 10 bis 30% des gesamten Porenvolumens, insbesondere 12 bis 20%.

Erfindungsgemäß weist das eingesetzte $TiO_2$ die folgende Teilchengrößenverteilung auf: Der $D_{50}$-Wert (d.h. der Wert, bei dem jeweils die Hälfte der Teilchen einen größeren bzw. kleineren Teilchendurchmesser aufweist) liegt bei 1,5 $\mu$m oder darunter und der $D_{90}$-Wert des eingesetzten $TiO_2$ liegt zwischen 0,5 und 20 $\mu$m.

Nach einer bevorzugten Ausführungsform liegt der $D_{10}$-Wert vorzugsweise bei 0,5 $\mu$m oder darunter, der $D_{90}$-Wert liegt vorzugsweise bei 4 $\mu$m oder darunter. Bevorzugt liegt der $D_{90}$-Wert des eingesetzten $TiO_2$ zwischen etwa 1 und 10 $\mu$m, besonders bevorzugt zwischen etwa 2 und 5 $\mu$m. Das Titandioxid liegt vorzugsweise in der Anatasform vor.

Erfindungsgemäß geeignete $TiO_2$-Materialien sind kommerziell erhältlich, z.B. unter den Handelsnamen NT22-B20 und NT22-B30 von der Firma Nano Inc., Ltd., 1108-1 Bongkok Sabong, Jinju, Kyoungnam 660-882 Korea) .

Auch ist dem Fachmann bekannt, dass sich die Primärkristallitgröße durch Tempern bzw. Calcinieren des $TiO_2$ vergrößern lässt. Beispielsweise kann eine Calcinierung in einem Drehrohrofen bei etwa 600°C für 24 bis 48 h in einer Mischung aus 50% Wasserdampf und 50% Luft erfolgen, um die Primärkristallitgröße zu erhöhen. Soweit die erfindungsgemäß vorausgesetzte Primärkristallitgröße noch nicht erreicht ist, kann die Prozedur wiederholt werden. Da gleichzeitig auch die BET-Oberfläche sinken kann, sollte von einem $TiO_2$-Material mit relativ hoher BET- Oberfläche ausgegangen werden, damit abschließend eine BET-Oberfläche von mehr als 15 $m^2/g$ vorliegt. Je nach vorgesehener Verwendung des erfindungsgemäßen Katalysators können neben dem erfindungsgemäß eingesetzten $TiO_2$, die dem Fachmann geläufigen und üblichen Komponenten in der aktiven Masse des Katalysators enthalten sein. Nach einer möglichen erfindungsgemäßen Ausführungsform kann auch nur ein Teil des zur Katalysatorherstellung verwendeten Titandioxids, die hierin beschriebenen Eigenschaften aufweisen, obwohl dies in der Regel nicht bevorzugt ist. Auch die Form des Katalysators bzw. dessen homogener oder heterogener Aufbau ist im Sinne der vorliegenden Erfindung grundsätzlich nicht beschränkt und kann jegliche dem Fachmann geläufige und für das jeweilige Anwendungsgebiet geeignet erscheinende Ausführungsform umfassen.

Soweit der erfindungsgemäße Katalysator nach einer besonders bevorzugten Ausführungsform zur Herstellung von Phthalsäureanhydrid eingesetzt wird, haben sich sogenannte Schalenkatalysatoren bewährt. Hierbei wird ein unter den Reaktionsbedingungen inerter Träger, beispielsweise aus Quarz ($SiO_2$), Porzellan, Magnesiumoxid, Zinndioxid,

Siliciumcarbid, Rutil, Tonerde (Al$_2$O$_3$), Aluminiumsilicat, Magnesiumsilicat (Steatit), Zirkoniumsilicat oder Cersilicat oder aus Mischungen der vorstehenden Materialien verwendet. Der Träger kann beispielsweise die Form von Ringen, Kugeln, Schalen oder Hohlzylindern aufweisen. Darauf wird in verhältnismäßig dünnen Schichten (Schalen) die katalytisch aktive Masse aufgebracht. Es können auch zwei oder mehrere Schichten der gleichen oder unterschiedlich zusammengesetzter katalytisch aktiver Masse aufgebracht werden.

Bezüglich der weiteren Komponenten der katalytisch aktiven Masse des erfindungsgemäßen Katalysators (neben TiO$_2$) kann grundsätzlich auf die im einschlägigen Stand der Technik beschriebenen und dem Fachmann geläufigen Zusammensetzungen bzw. Komponenten verwiesen werden. Dabei handelt es sich hauptsächlich um Katalysatorsysteme, die neben Titanoxid(en) Oxide des Vanadiums enthalten. Solche Katalysatoren sind z.B. in der EP 0 964 744 B1 beschrieben, deren diesbezüglich Offenbarung hiermit ausdrücklich durch Inbezugnahme in die Beschreibung aufgenommen wird.

Insbesondere sind im Stand der Technik eine Reihe von Promotoren zur Steigerung der Produktivität der Katalysatoren beschrieben, die im erfindungsgemäßen Katalysator ebenfalls eingesetzt werden können. Dazu gehören u.a. die Alkali- und Erdalkalimetalle, Thallium, Antimon, Phosphor, Eisen, Niob, Kobalt, Molybdän, Silber, Wolfram, Zinn, Blei und/oder Bismut sowie Mischungen aus zwei oder mehreren der vorstehenden Komponenten. Beispielsweise ist in der DE 21 59 441 A ein Katalysator beschrieben, der neben Titandioxid der Anatas-Modifikation aus 1 bis 30 Gew.-% Vanadiumpentoxid und Zirkondioxid besteht. Über die einzelnen Promotoren lässt sich die Aktivität und Selektivität der Katalysatoren beeinflussen, insbesondere durch Absenkung oder Erhöhung der Aktivität. Zu den die Selektivität erhöhenden Promotoren zählen beispielsweise die Alkalimetalloxide, wohingegen oxidische Phosphorverbindungen, insbesondere Phosphorpentoxid, die Aktivität des Katalysators auf Kosten der Selektivität erhöhen.

Zur Herstellung der erfindungsgemäßen Katalysatoren sind im Stand der Technik zahlreiche geeignete Verfahren beschrieben, so dass eine detaillierte Darstellung hier grundsätzlich nicht erforderlich ist. Zur Herstellung von Schalenkatalysatoren kann beispielsweise auf das in der DE 16 42938 A1 oder der DE 1769998 A1 beschriebene Verfahren verwiesen werden, worin eine ein wässriges und/oder ein organisches Lösungsmittel enthaltende Lösung oder Suspension der Komponenten der katalytisch aktiven Masse und/oder deren Vorläuferverbindungen (häufig als "Maische" bezeichnet) auf das Trägermaterial in einer beheizten Dragiertrommel bei erhöhter Temperatur aufgesprüht werden, bis der gewünschte Gehalt an katalytisch aktiver Masse, bezogen auf das Katalysatorgesamtgewicht, erreicht ist. Auch lässt sich gemäß der DE 2106796 A1 die Aufbringung (Beschichtung) der katalytisch aktiven Masse auf den inerten Träger in Wirbelbeschichtern durchführen.

Bevorzugt werden sogenannte Schalenkatalysatoren durch das Aufbringen einer dünnen Schicht von 50 bis 500 $\mu$m der Aktivkomponenten auf einen inerten Träger hergestellt (z.B. US 2,035,606). Als Träger haben sich insbesondere Kugeln oder Hohlzylinder bewährt. Diese Formkörper ergeben eine hohe Packungsdichte bei niedrigem Druckverlust und verringern die Gefahr der Bildung von Packungsfehlern beim Einfüllen des Katalysators in die Reaktionsrohre.

Die geschmolzenen und gesinterten Formkörper müssen innerhalb des Temperaturbereiches der ablaufenden Reaktion hitzebeständig sein. Wie vorstehend ausgeführt, kommen dabei beispielsweise Siliciumcarbid, Steatit, Quarz, Porzellan, SiO$_2$, Al$_2$O$_3$ oder Tonerde in Frage.

Der Vorteil der Beschichtung von Trägerkörpern im Wirbelbett ist die hohe Gleichmäßigkeit der Schichtdicke, die für die katalytische Leistung des Katalysators eine entscheidende Rolle spielt. Eine besonders gleichmäßige Beschichtung erhält man durch Aufsprühen einer Suspension oder Lösung der Aktivkomponenten auf den erwärmten Träger bei 80 bis 200°C im Wirbelbett, beispielsweise gemäß DE 12 80 756 B, DE 198 28 583 A1 oder DE 197 09 589 A1. Im Gegensatz zu der Beschichtung in Dragiertrommeln kann bei Verwendung von Hohlzylindern als Träger in den genannten Wirbelbettverfahren auch die Innenseite der Hohlzylinder gleichmäßig beschichtet werden. Unter den oben genannten Wirbelbettverfahren ist insbesondere das Verfahren nach DE 197 09 589 A1 von Vorteil, da durch die überwiegend horizontale, kreisförmige Bewegung der Träger neben einer gleichmäßigen Beschichtung auch eine geringe Abrasion von Apparateteilen erreicht wird.

Für den Beschichtungsvorgang wird die wässrige Lösung oder Suspension der Aktivkomponenten und eines organischen Binders, vorzugsweise einem Copolymer aus Vinylacetat/Vinyllaurat, Vinylacetat/Ethylen oder Styrol/Acrylat, über eine oder mehrere Düsen auf den erwärmten, fluidisierten Träger aufgesprüht. Besonders günstig ist es, die Sprühflüssigkeit am Ort der höchsten Produktgeschwindigkeit aufzugeben, wodurch sich der Sprühstoff gleichmäßig im Bett verteilen kann. Der Sprühvorgang wird solange fortgeführt, bis entweder die Suspension verbraucht oder die erforderliche Menge an Aktivkomponenten auf dem Träger aufgebracht ist.

Nach einer besonders bevorzugten erfindungsgemäßen Ausführungsform wird die katalytisch aktive Masse des erfindungsgemäßen Katalysators, enthaltend das TiO$_2$ wie hierin definiert, im Fließbett oder Wirbelbett unter Beihilfe geeigneter Bindemittel aufgebracht, so dass ein Schalenkatalysator erzeugt wird. Geeignete Bindemittel umfassen dem Fachmann geläufige organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat sowie Vinylacetat/Ethylen. Besonders bevorzugt wird ein organischer polymerer oder copolymerer Kleber, insbesondere ein Vinylacetat-Copolymer-Kleber, als Bindemittel verwendet. Das verwendete Bindemittel wird in üblichen Mengen der katalytisch aktiven Masse zugegeben, beispielsweise mit etwa 10 bis 20 Gew.-%, bezogen auf den Feststoffgehalt der katalytisch aktiven Masse. Beispielsweise kann auf die EP 744 214 A1 verwiesen werden. Soweit die Aufbringung der katalytisch aktiven Masse bei erhöhten Temperaturen von etwa 150°C erfolgt, ist, wie aus dem Stand der Technik bekannt, eine Aufbringung auf den Träger auch ohne organische Bindemittel möglich. Brauchbare Beschichtungstemperaturen bei Verwendung der vorstehend angegebenen Bindemittel liegen gemäß DE 21 06 796 A1 beispielsweise zwischen etwa 50 und 450°C. Die verwendeten Bindemittel brennen beim Ausheizen des Katalysators bei Inbetriebnahme des gefüllten Reaktors innerhalb kurzer Zeit aus. Die Bindemittel dienen in erster Linie der Verstärkung der Haftung der katalytisch aktiven Masse auf dem Träger und der Verringerung von Abrieb beim Transport und Einfüllen des Katalysators.

Weitere mögliche Verfahren zur Herstellung von Schalenkatalysatoren für die katalytische Gasphasenoxidation von aromatischen Kohlenwasserstoffen zu Carbonsäuren und/oder Carbonsäureanhydriden sind beispielsweise in der WO 98/00778 A1 bzw. EP 714700 A2 beschrieben worden. Danach wird aus einer Lösung und/oder einer Suspension der katalytisch aktiven Metalloxide und/oder deren Vorläuferverbindungen, gegebenenfalls in Anwesenheit von Hilfsmitteln für die Katalysatorherstellung, zunächst ein Pulver hergestellt, das anschließend für die Katalysatorherstellung auf dem Träger, gegebenenfalls nach Konditionierung sowie gegebenenfalls nach Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide oder einer Behandlung zur Entfernung flüchtiger Bestandteile unterzogen.

Geeignete Bedingungen zur Durchführung eines Verfahrens zur Gasphasenoxidation von Kohlenwasserstoffen, insbesondere zur Herstellung von Phthalsäureanhydrid aus o-Xylol und/oder Naphthalin sind dem Fachmann gleichermaßen aus dem Stand der Technik bekannt. Insbesondere wird auf die zusammenfassende Darstellung in K. Towae, W. Enke, R. Jäckh, N. Bhargana "Phtalic Acid and Derivatives" in Ullmann' s Encyclopedia of Industrial Chemistry Vol. A. 20, 1992, 181 verwiesen und diese hiermit durch Bezugnahme aufgenommen. Beispielsweise können für den stationären Betriebszustand der Oxidation die aus der vorstehenden Literaturstelle der WO98/37967 A1 oder der WO 99/61433 A1 bekannten Randbedingungen gewählt werden.

Dazu werden zunächst die Katalysatoren in die Reaktionsrohre des Reaktors, die von außen auf die Reaktionstemperatur, beispielsweise mittels Salzschmelzen thermostatisiert sind, gefüllt. Über die so bereitete Katalysatorschüttung wird das Reaktionsgas bei Temperaturen von im Allgemeinen 300 bis 450°C, vorzugsweise 320 bis 420°C, und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von im Allgemeinen 750 bis 5000 h$^{-1}$ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel wie Dampf, Kohlendioxid und/oder Stickstoff enthalten kann, mit dem zu oxidierenden, aromatischen Kohlenwasserstoff erzeugt, wobei das den molekularen Sauerstoff enthaltende Gas im Allgemeinen 1 bis 100, vorzugsweise 2 bis 50 und besonders bevorzugt 10 bis 30 mol-% Sauerstoff, 0 bis 30, vorzugsweise 0 bis 10 mol-% Wasserdampf sowie 0 bis 50, vorzugsweise 0 bis 1 mol-% Kohlendioxid, Rest Stickstoff, enthalten kann. Zur Erzeugung des Reaktionsgases wird das den molekularen Sauerstoff enthaltende Gas im Allgemeinen mit 30 bis 150 g je Nm$^3$ Gas des zu oxidierenden, aromatischen Kohlenwasserstoffs beschickt.

Nach einer besonders bevorzugten erfindungsgemäßen Ausführungsform weist der erfindungsgemäße Katalysator einen Aktivmassengehalt zwischen etwa 7 und 12 Gew.-%, insbesondere zwischen 8 und 10 Gew.-% auf, wobei die Aktivmasse (katalytisch aktive Masse) zwischen 5 bis 15 Gew.-% $V_2O_5$, 0 bis 4 Gew. - $Sb_2O_3$, 0,2 bis 0,75 Gew.-% Cs, 0 bis 3 Gew.-% $Nb_2O_5$ enthält. Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$. Ein solcher erfindungsgemäßer Katalysator kann als solcher, oder beispielsweise bei einem Zwei- oder Mehrlagen-Katalysator als erste, zur Gaseintrittsseite hin gelegene Katalysatorlage verwendet werden.

Nach einer besonders bevorzugten erfindungsgemäßen Ausführungsform liegt dabei die BET-Oberfläche des Katalysators zwischen 15 und etwa 25 m$^2$/g. Weiterhin wird bevorzugt, dass eine solche erste Katalysatorlage einen Längenanteil von etwa 40 bis 60% an der Gesamtlänge aller vorhandenen Katalysatorlagen (Gesamtlänge des vorhandenen Katalysatorbettes) aufweist.

Nach einer weiteren bevorzugten erfindungsgemäßen Ausführungsform weist der erfindungsgemäße Katalysator einen Aktivmassengehalt von etwa 6 bis 11 Gew.-%, insbesondere 7 bis 9 Gew.-% auf, wobei die aktive Masse 5 bis 15 Gew.-% $V_2O_5$, 0 bis 4 Gew.-% $Sb_2O_3$, 0,05 bis 0,3 Gew.-% Cs, 0 bis 2 Gew.-% $Nb_2O_5$ enthält. Neben den vorstehenden Komponenten besteht der Rest der Aktivmasse zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew-% aus $TiO_2$. Ein solcher erfindungsgemäßer Katalysator kann beispielsweise als zweite Katalysatorlage, d.h. stromab der zur Gaseintrittsseite hin gelegenen ersten Katalysatorlage (vgl. oben) eingesetzt werden. Dabei wird bevorzugt, dass der Katalysator eine BET-Oberfläche zwischen etwa 15 und 25 m$^2$/g aufweist. Weiterhin wird bevorzugt, dass diese zweite Lage einen Längenanteil von etwa 10 bis 30% der Gesamtlänge aller vorhandenen Katalysatorlagen einnimmt.

Nach einer weiteren erfindungsgemäßen Ausführungsform weist der erfindungsgemäße Katalysator einen Aktivmassengehalt zwischen etwa 5 und 10 Gew.-%, insbesondere zwischen 6 und 8 Gew.-% auf, wobei die Aktivmasse (katalytisch aktive Masse) 5 bis 15 Gew.-% $V_2O_5$, 0 bis 4 Gew.-% $Sb_2O_3$, 0 bis 0,1 Gew.-% Cs, 0 bis 1 Gew.-% $Nb_2O_5$ enthält. Neben den vorstehenden Komponenten besteht der Rest der Aktivmasse zu mindestens 90 Gew.-%, vorzugsweise mindestens 95 Gew.-%, weiter bevorzugt mindestens 98 Gew.-%, insbesondere mindestens 99 Gew.-%, weiter bevorzugt mindestens 99,5 Gew.-%, insbesondere 100 Gew.-% aus $TiO_2$. Bevorzugt wird dabei eine BET-Oberfläche des Katalysators, die etwas höher liegt als diejenige der näher zur Gaseintrittsseite hin gelegenen Schichten, insbesondere im Bereich zwischen etwa 25 bis etwa 45 $m^2/g$. Weiterhin wird bevorzugt, dass eine solche dritte Katalysatorlage einen Längenanteil von etwa 10 bis 50% der Gesamtlänge aller vorhandenen Katalysatorlagen einnimmt.

Das Temperaturmanagement bei der Gasphasenoxidation von o-Xylol zu Phthalsäureanhydrid ist dem Fachmann aus dem Stand der Technik hinreichend bekannt, wobei beispielsweise auf die DE 10040827 A1 verwiesen werden kann.

Es wurde gefunden, dass nach einer bevorzugten Ausführungsform erfindungsgemäß Katalysatoren, die kein Phosphor in der katalytisch aktiven Masse aufweisen, im Zusammenspiel mit dem erfindungsgemäß eingesetzten $TiO_2$ besonders gute Aktivitäten bei gleichzeitig sehr hoher Selektivität ermöglichen. Dabei wird weiterhin bevorzugt, dass mindestens 0,05 Gew.-% der katalytisch aktiven Masse durch mindestens ein Alkalimetall, berechnet als Alkalimetall (e) gebildet wird. Besonders bevorzugt wird als Alkalimetall Cäsium verwendet.

Zudem wird nach den Ergebnissen der Erfinder nach einer Ausführungsform bevorzugt, dass der erfindungsgemäße Katalysator Niob in einer Menge von 0,01 bis 2 Gew.-%, insbesondere 0,5 bis 1 Gew.-% der katalytisch aktiven Masse enthält.

Die erfindungsgemäßen Katalysatoren werden in üblicher Weise vor dem Einsatz temperaturbehandelt bzw. calciniert (konditioniert). Dabei hat sich als vorteilhaft herausgestellt, wenn der Katalysator mindestens 24 Stunden bei mindestens 390°C, insbesondere zwischen 24 und 72 Stunden bei mindestens 400°C, in einem $O_2$- haltigen Gas, insbesondere in Luft, calciniert wird. Die Temperaturen sollten vorzugsweise etwa 500°C, insbesondere etwa 470°C, nicht übersteigen. Grundsätzlich sind jedoch auch andere Calcinierungsbedingungen, die dem Fachmann als geeignet erscheinen, nicht ausgeschlossen. Nach einem weiteren Aspekt betrifft die vorliegende Erfindung ein Verfahren zur Herstellung eines Katalysators gemäß einem der vorstehenden Ansprüche, umfassend die folgenden Schritte:

a. Bereitstellen einer katalytisch aktiven Masse wie hierin definiert, enthaltend das vorstehend näher charakterisierte $TiO_2$;

b. Bereitstellen eines inerten Trägers, insbesondere eines inerten Trägerformkörpers;

c. Aufbringen der katalytisch aktiven Masse auf den inerten Träger, insbesondere in einer Wirbelschicht oder einem Fließbett.

Nach einem weiteren Aspekt betrifft die vorliegende Erfindung auch die Verwendung eines Titandioxids wie vorstehend definiert zur Herstellung eines Katalysators, insbesondere zur Gasphasenoxidation von Kohlenwasserstoffen, vorzugsweise zur Gasphasenoxidation von o-Xylol und/oder Naphthalin zu Phthalsäureanhydrid.

METHODEN

Zur Bestimmung der Parameter der erfindungsgemäßen Katalysatoren werden die nachstehenden Methoden eingesetzt:

1. BET-Oberflache:

Die Bestimmung erfolgt nach der BET-Methode gemäß DIN 66131; eine Veröffentlichung der BET-Methode findet sich auch in J. Am. Chem. Soc . 60, 309 (1938) .

2. Porenradienverteilung:

Die Bestimmung der Porenradienverteilung und des Porenvolumens des eingesetzten $TiO_2$ erfolgte mittels Quecksilberporosimetrie gemäß DIN 66133; maximaler Druck: 2.000 bar, Porosimeter 4000 (Firma Porotec, DE), nach Angaben des Herstellers.

3. Primärkristallitgrößen:

Die Bestimmung der Primärkristallitgrößen (Primärpartikelgrößen) erfolgte mittels Pulver-Röntgendiffraktometrie. Die Analyse wurde mit einem Gerät der Firma Bruker, DE, durchgeführt: Typ BRUKER AXS - D4 Endeavor. Die erhaltenen Röntgendiffraktogramme wurden mit dem Softwarepaket "DiffracPlus D4 Measurement" gemäß den Angaben des Herstellers aufgezeichnet und

die Halbwertsbreite des 100% Reflexes wurde mit der Software "DiffracPlus Evaluation" nach der Debye-Scherrer Formel gemäß den Angaben des Herstellers ausgewertet, um die Primärkristallitgröße zu bestimmen.

4. Teilchengrößen:

Die Bestimmung der Teilchengrößen erfolgte nach der Laserbeugungsmethode mit einem Fritsch Particle Sizer Analysette 22 Economy (Fa. Fritsch, DE) nach den Angaben des Herstellers, auch bezüglich der Probenvorbehandlung: die Probe wird in deionisiertem Wasser ohne Zusatz von Hilfsmitteln homogenisiert und 5 Minuten mit Ultraschall behandelt.

5. Alkaligehalt des $TiO_2$

Der Alkaligehalt des $TiO_2$ wird nach DIN ISO 9964-3 bestimmt. So kann Alkali mittels ICP-AES (Inductively Coupled Plasma Atomic Emission Spectroscopy) bestimmt und ggf. zum Gesamtalkaligehalt des $TiO_2$ aufaddiert werden.

6. Schüttdichte:

Die Schüttdichte wurde anhand des zur Herstellung des Katalysators eingesetzten $TiO_2$ (bei 150°C im Vakuum getrocknet, uncalciniert) bestimmt. Die erhaltenen Werte aus drei Bestimmungen wurden gemittelt. Die Schüttdichte wurde bestimmt, indem 100 g des $TiO_2$- Materials in eine 1.000 ml-Dose eingefüllt und ca. 30 Sekunden geschüttelt wurden.

Ein Messzylinder (Fassungsvermögen genau 100ml) wird leer auf 10 mg gewogen. Darauf wird der Pulvertrichter mit Stativ und Klemme über die Öffnung des Zylinders befestigt. Nach Ingangsetzung der Stoppuhr wird der Messzylinder innerhalb von 15 Sekunden mit dem $TiO_2$-Material gefüllt. Mit dem Spatel wird laufend Füllgut nachgeschüttet, so dass der Messzylinder immer leicht überstehend gefüllt ist. Nach 2 Minuten wird mit dem Spatel der Überstand abgestreift, wobei darauf zu achten ist, dass keine Presskräfte das Material im Zylinder verdichten. Der gefüllte Messzylinder wird abgepinselt und gewogen.
Die Schüttdichte wird in g/l angegeben.
Die Bestimmung der BET-Oberfläche, der Porenradienverteilung bzw. des Porenvolumens sowie der Primärkristallitgrößen und der Teilchengrößenverteilung erfolgte bezüglich des Titandioxids jeweils an dem bei 150°C im Vakuum getrockneten, uncalcinierten Material.
Auch die Angaben in der vorliegenden Beschreibung bezüglich der BET-Oberflachen der Katalysatoren bzw. Katalysatorlagen beziehen sich auf die BET-Oberflachen des jeweils eingesetzten $TiO_2$-Materials (getrocknet in Vakuum bei 150°C, uncalciniert, vgl. oben).
In der Regel wird die BET-Oberfläche des Katalysators durch die BET-Oberfläche des eingesetzten $TiO_2$ bestimmt, wobei durch den Zusatz weiterer katalytisch aktiver Komponenten die BET-Oberfläche in gewissem Umfang verändert wird. Dies ist dem Fachmann geläufig. Der Aktivmasseanteil (Anteil der katalytisch aktiven Masse, ohne Bindemittel) bezieht sich jeweils auf den Anteil (in Gew.-%) der katalytisch aktiven Masse an dem Gesamtgewicht des Katalysators einschließlich Träger in der jeweiligen Katalysatorlage, gemessen nach Konditionierung über 4h bei 400°C.
Die Erfindung wird nun anhand der nachstehenden, nicht beschränkenden Beispiele näher erläutert:

BEISPIELE

Beispiel 1: Herstellung von Katalysator A (Vergleich 1)

Zur Herstellung des Katalysators A mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid (Fa. Sachtleben Chemie GmbH, Duisburg, Handelname: Hombikat T; Lot. Nr. E3-588-352-001) wurden in einem sogenannten Wirbelbett-Coater 2600 g Steatitkörper in Form von Hohl-Zylindern der Größe 8 x 6 x 5 mm mit einer Suspension aus 17,9 g Vanadiumpentoxid, 7,6 g Antimontrioxid, 1,28 g Cäsiumsulfat, 1,9 g Ammoniumdihydrogenphosphat, 364,4 g Titandioxid, 130,5 g Bindemittel aus einer 50%-igen Dispersion von Wasser und Vinylacetat/Ethylencopolymer (Vinnapas® EP 65 W, Fa. Wacker) und 1000 g Wasser bei einer Temperatur von 70 °C beschichtet. Die Aktivmasse wurde in Form dünner Schichten aufgetragen.
Das Titandioxid wies eine BET-Oberfläche von 26 m$^2$/g, eine Schüttdichte von 1,23 g/ml, eine Primärkristallitgröße von 200 Angström, eine Porenradienverteilung von 50 % des gesamten Porenvolumens durch Poren mit einem Radius von 7500 bis 400 nm

1,7 % des gesamten Porenvolumens durch Poren mit einem Radius von 400 bis 60 nm

48 % des gesamten Porenvolumens durch Poren mit einem Radius von 60 bis 3,7 nm,

sowie eine Teilchengrößenverteilung von $D_{10}$ = 12,4 $\mu$m, $D_{50}$ = 31,6 $\mu$m, $D_{90}$ = 64,7 $\mu$m

sowie einen Gesamtalkaligehalt (Li + Na + K + Rb + Cs) von mehr als 2000 ppm.

auf.

Beispiel 2: Herstellung von Katalysator B (Vergleich 2)

Zur Herstellung des Katalysators B mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,1 g Vanadiumpentoxid, 6,4 g Antimontrioxid, 1,08 g Cäsiumcarbonat, 1,5 g Ammoniumdihydrogenphosphat, 178,62 g Titandioxid, 130,5 g Bindemittel (siehe Beispiel 1) und 2000 g Wasser bei einer Temperatur von 70 °C beschichtet. Die Aktivmasse wurde in Form dünner Schichten aufgetragen.

Das Titandioxid aus Beispiel 1 wurde hierzu in 0,1 M wässriger $HNO_3$ aufgeschlämmt und unter Rückfluss und unter Agitation bei 90°C über Nacht gewaschen, anschließend filtriert und dreifach mit bidestilliertem Wasser gewaschen und bei 150°C an Luft getrocknet. Das Titandioxid wies danach eine BET- Oberfläche von 24,3 m$^2$/g, eine Schüttdichte von 1,09 g/ml, eine Primärkristallitgröße von 200 Angström, eine Porenradienverteilung von

52 % des gesamten Porenvolumens durch Poren mit einem Radius von 7500 bis 400 nm

4,7 % des gesamten Porenvolumens durch Poren mit einem Radius von 400 bis 60 nm

43 % des gesamten Porenvolumens durch Poren mit einem Radius von 60 bis 3,7 nm,

sowie eine Teilchengrößenverteilung von $D_{10}$ = 9,8 $\mu$m, D50 = 32,5 $\mu$m, $D_{90}$ = 65,1 $\mu$m

sowie einen Gesamtalkaligehalt (Li + Na + K + Rb + Cs) von weniger als 1000 ppm

auf.

Beispiel 3 : Herstellung von Katalysator C (Vergleich 3)

Zur Herstellung des Katalysators C mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 17 g Vanadiumpentoxid, 7,03 g Antimontrioxid, 1,14 g Cäsiumsulfat, 1,7 g Ammoniumdihydrogenphosphat, 195,0 g Titandioxid, 130,5 g Bindemittel (siehe Beispiel 1) und 2000 g Wasser bei einer Temperatur von 70 °C beschichtet. Die Aktivmasse wurde in Form dünner Schichten aufgetragen.

Das Titandioxid (Firma Nano Inc., Ltd., 1108-1 Bongkok Sabong, Jinju, Kyoungnam 660-882 Korea, Handelsname NT22-B20) wies eine BET-Oberfläche von 18 m$^2$/g, eine Schüttdichte von 0,48 g/ml, eine Primärkristallitgröße von 390 Angström, und eine Porenradienverteilung von

-   43 % des gesamten Porenvolumens durch Poren mit einem Radius von 7500 bis 400 nm

-   47 % des gesamten Porenvolumens durch Poren mit einem Radius von 400 bis 60 nm

10 % des gesamten Porenvolumens durch Poren mit einem Radius von 60 bis 3,7 nm,

sowie eine Teilchengrößenverteilung von $D_{10}$ = 0,4 $\mu$m, $D_{50}$ = 1,2 $\mu$m, $D_{90}$ = 2,8 $\mu$m

und einen Gesamtalkaligehalt von weniger als 1000 ppm auf.

Beispiel 4 : Herstellung von Katalysator D (Erfindungsgemäß)

Zur Herstellung des Katalysators D mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 17 g Vanadiumpentoxid, 7,03 g Antimontrioxid, 1,14 g Cäsiumsulfat, 1,7 g Ammoniumdihydrogenphosphat, 195,0 g Titandioxid, 130,5 g Bindemittel (siehe Beispiel 1) und 2000 g Wasser bei einer Temperatur von 70 °C beschichtet. Die Aktivmasse wurde in Form dünner Schichten aufgetragen.

Das Titandioxid (Fa. Nano Inc. Ltd., siehe oben, Handelsname NT22-B30) mit einer BET-Oberfläche von 34 m²/g, wurde in einem Drehrohrofen bei 600 °C für 48h in einer Mischung aus 50% Wasserdampf und 50% Luft behandelt. Nach dieser Temperaturbehandlung wies das Titanoxid eine BET-Oberfläche von 24 m²/g, eine Schüttdichte von 0,47 g/ml, eine Primärkristallitgröße von 349 Angström, und eine Porenradienverteilung von

19 % des gesamten Porenvolumens durch Poren mit einem Radius von 7500 bis 400 nm
66 % des gesamten Porenvolumens durch Poren mit einem Radius von 400 bis 60 nm
16 % des gesamten Porenvolumens durch Poren mit einem Radius von 60 bis 3,7 nm,
sowie eine Teilchengrößenverteilung von $D_{10}$ = 0,4 μm, $D_{50}$ = 1,4 μm, $D_{90}$ = 16,9 μm
und einen Gesamtalkaligehalt von weniger als 1000 ppm
auf.

Beispiel 5: Ermittlung der katalytischen Leistungsdaten von Katalysator A (Vergleich 1)

In ein 120 cm langes Reaktionsrohr mit einem Innendurchmesser von 24,8 mm werden 40 g Katalysator A, verdünnt mit 200 g Steatitringen der Abmessungen 8 x 6 x 5 mm zur Vermeidung von Hotspots, auf einer Länge von 80 cm eingefüllt. Das Reaktionsrohr befindet sich in einer flüssigen Salzschmelze die auf Temperaturen bis 450 °C aufgeheizt werden kann. In der Katalysatorschüttung befindet sich ein 3 mm Schutzrohr mit eingebautem Thermoelement über das die Katalysatortemperatur über die komplette Katalysatorkombination angezeigt werden kann. Zur Ermittlung der katalytischen Leistungsdaten werden über den Katalysator A 60 g/Nm³ o-Xylol (Reinheit 99,9%) bei maximal 400 NI Luft/h geleitet, so dass eine katalysatormassebezogene Raumgeschwindigkeit von 5,12 l/h x $m_{kat}$ bei einer mittleren Katalysatortemperatur von 420°C eingestellt wird, und das Reaktionsgas nach Reaktionsrohraustritt auf seine Bestandteile hin analysiert. Die Ergebnisse des Testlaufs sind in der Tabelle 1 aufgeführt.

Beispiel 6: Ermittlung der katalytischen Leistungsdaten von Katalysator B (Vergleich 2)

In ein 120 cm langes Reaktionsrohr mit einem Innendurchmesser von 24,8 mm werden 40 g Katalysator B, verdünnt mit 200 g Steatitringen der Abmessungen 8 x 6 x 5 mm zur Vermeidung von Hotspots, auf einer Länge von 80 cm eingefüllt. Ansonsten verfährt man wie unter Beispiel 5 beschrieben. Die Ergebnisse des Testlaufs sind in der Tabelle 1 aufgeführt.

Beispiel 7 : Ermittlung der katalytischen Leistungsdaten von Katalysator C (Vergleich 3) In ein 120 cm langes Reaktionsrohr mit einem Innendurchmesser von 24 , 8 mm werden 40 g Katalysator B, verdünnt mit 200 g Steatitringen der Abmessungen 8 x 6 x 5 mm zur Vermeidung von Hotspots , auf einer Länge von 80 cm eingefüllt. Ansonsten verfährt man wie unter Beispiel 5 beschrieben.

Beispiel 8 : Ermittlung der katalytischen Leistungsdaten von Katalysator D (Erfindungsgemäß)

In ein 120 cm langes Reaktionsrohr mit einem Innendurchmesser von 24,8 mm werden 40 g Katalysator B, verdünnt mit 200 g Steatitringen der Abmessungen 8 x 6 x 5 mm zur Vermeidung von Hotspots , auf einer Länge von 80 cm eingefüllt. Ansonsten verfährt man wie unter Beispiel 5 beschrieben.

Die Ergebnisse des Testlaufs sind in der Tabelle 1 aufgeführt.

Tabelle 1 : Auflistung der Versuchsergebnisse

| Beispiel | Umsatz [%] | $C_8$-Selektivität [mol.-%] | Selektivität PSA [mol.-%] | Selektivität $CO_x$ [mol.-%] |
|---|---|---|---|---|
| Katalysator A (Bsp. 5) | 26 | 55,7 | 32,2 | 39,1 |
| Katalysator B (Bsp. 6) | 55,3 | 73,7 | 52,2 | 21,3 |
| Katalysator C (Bsp. 7) | 72,4 | 86,3 | 71,2 | 10,1 |
| Katalysator D (Bsp. 8) | 95,3 | 85,6 | 81,9 | 11,5 |

$C_8$-Selektivität : Selektivität bzgl. aller Wertprodukte mit 8 Kohlenstoffatomen (Phthalsäureanhydrid, Phthalid, o-Tolylaldehyd, o-Tolylsäure) $CO_x$ : Summe aus Kohlenmonoxid und -dioxid im Abgasstrom

Beispiel 9 : Herstellung eines erfindungsgemäßen Dreischicht-Katalysators

Ein erfindungsgemäßer Dreischicht -Katalysator kann beispielsweise wie folgt erhalten werden: - -

Zur Herstellung eines Katalysators E mit einem Aktivmassenanteil von 9 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 17,0 g Vanadiumpentoxid, 7,0 g Antimontrioxid, 1,1 g Cäsiumsulfat, 1,65 g Ammoniumdihydrogenphosphat, 194,9 g Titandioxid mit einer BET-Oberfläche von 18 m$^2$/g (wie in Beispiel 3), 102,1 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung eines Katalysators F mit einem Aktivmassenanteil von 8 Gew.-% und der Zusammensetzung von 7,5 Gew.-% Vanadiumpentoxid, 3,2 Gew.-% Antimontrioxid, 0,20 Gew.-% Cäsium (berechnet als Cäsium), 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 15,1 g Vanadiumpentoxid, 6,3 g Antimontrioxid, 0,53 g Cäsiumsulfat, 1,47 g Ammoniumdihydrogenphosphat, 173,7 g Titandioxid mit einer BET-Oberfläche von 18 m$^2$/g (wie in Beispiel 3), 101 g Bindemittel aus einer 50%- igen Dispersion von Wasser und Vinylacetat/Ethylencopolymer (Vinnapas EP 65 W, Fa. Wacker) und 2000 g Wasser bei einer Temperatur von 70°C beschichtet. Die aktive Masse wurde in Form dünner Schichten aufgetragen.

Zur Herstellung eines Katalysators G mit einem Aktivmassenanteil von 8 Gew.-% und der Zusammensetzung von 7,5 Gew.-% Vanadiumpentoxid, 3,2 Gew.-% Antimontrioxid, 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 15,1 g Vanadiumpentoxid, 6,25 g Antimontrioxid, 1,47 g Ammoniumdihydrogenphosphat, 174,11 g Titandioxid mit einer BET-Oberfläche von 27 m$^2$/g (Mischung aus NT22-B20 (siehe Beispiel 3) und NT22-B30 (siehe Beispiel 4, ohne Calcinierung), 101 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen. Die Schüttdichte des TiO$_2$ lag für die Katalysatoren E, F und G jeweils bei unter 0,5 g/ml, die Primärkristallitgröße bei über 340 Angström; mindestens 25% des gesamten Porenvolumens werden durch Poren mit einem Radius zwischen 60 und 400 nm gebildet.

Die Abfolge der Katalysatorlagen: 160 cm des Katalysators E, 60 cm des Katalysators F, 70 cm des Katalysators G.

Beispiel 10 : Katalytische Leistungsdaten des erfindungsgemäßen Dreischicht-Katalysators

In ein 450 cm langes Reaktionsrohr werden hintereinander 70 cm des Katalysators G, 60 cm des Katalysators F und 160 cm des Katalysators E gefüllt. Das Reaktionsrohr befindet sich in einer flüssigen Salzschmelze, die auf Temperaturen bis 450°C aufgeheizt werden kann. In der Katalysatorschüttung befindet sich ein 3 mm Schutzrohr mit eingebauten Thermoelement, über das die Katalysatortemperatur über die komplette Katalysatorkombination angezeigt werden kann. Zur Ermittlung der katalytischen Leistungsdaten werden über diese Katalysatorkombination in der Reihenfolge DEF von 0 bis maximal 70 g/Nm$^3$ o-Xylol (Reinheit 99,9%) bei 3,6 Nm$^3$ Luft/h geleitet und das Reaktionsgas nach Reaktionsrohraustritt durch einen Kondensator geleitet, indem sich alle organischen Bestandteile des Reaktionsgases, bis auf das Kohlenmonoxid und Kohlendioxid, abscheiden. Das abgeschiedene Rohprodukt wird mittels überhitzten Dampf abgeschmolzen, aufgefangen und anschließend verwogen.

Die Rohausbeute wird wie folgt bestimmt.

Max. Roh-PSA-Ausbeute [Gew. -%]

$$\text{Ausgewogene Menge Roh-PSA [g]} \times 100 / \text{Zulauf o-Xylol [g]} \times \text{Reinheit o-Xylol [\%/100]}$$

Die Ergebnisse des Testlaufs sind in der Tabelle 2 aufgeführt.

Tabelle 2

| Beispiel | Maximal Beladung | Roh-PSA-Ausbeute | PSA-Qualität (Phthalidwert im Reaktionspas) | Hotspottemperatur und - lage |
|---|---|---|---|---|
| Beispiel 10: Katalysatorkombination E (160 cm) F (60 cm) G (70 cm) | 60 g/Nm$^3$ | 114,1 Gew.-% | < 500 ppm | 442 °C 55 cm (1. Lage) |

Wie aus Tabelle 2 ersichtlich, zeigt der erfindungsgemäße Katalysator gemäß Beispiel 9 eine sehr gute PSA-Ausbeute und PSA- Qualität. Der Hot Spot ist vorteilhaft in der ersten Katalysatorlage positioniert.

**Patentansprüche**

1. Katalysator, insbesondere zur Herstellung von Phthalsäureanhydrid durch Gasphasenoxidation von o-Xylol und/oder Naphthalin, mit einem inerten Träger und mindestens einer darauf aufgebrachten Schicht mit einer katalytisch aktiven Masse enthaltend TiO$_2$, **dadurch gekennzeichnet, dass** zumindest ein Teil des eingesetzten TiO$_2$ folgende Eigenschaften aufweist: (a) die BET-Oberfläche beträgt mehr als 15 m$^2$/g, (b) die Primärkristallitgröße, bestimmt mittels Pulver-Röntgendiffraktometrie nach der Debye-Scherrer-Formel, liegt bei mehr als 210 Angström und unter 900 Angström, wobei der D$_{90}$-Wert des eingesetzten TiO$_2$ zwischen 0,5 und 20 $\mu$m liegt, der D$_{50}$-Wert bei 1,5 $\mu$m oder darunter liegt und weniger als 22% des gesamten Porenvolumens des eingesetzten TiO$_2$ durch Poren mit einem Radius von mehr als 400 nm gebildet werden.

2. Katalysator nach Anspruch 1, **dadurch gekennzeichnet, dass** die Schüttdichte des eingesetzten TiO$_2$ bei weniger als 1,0 g/ml, vorzugsweise weniger als 0,8 g/ml, insbesondere weniger als 0,6 g/ml liegt.

3. Katalysator nach einem der vorstehenden Ansprüche, **dadurch gekennzeichnet, dass** zumindest ein Teil des eingesetzten TiO$_2$ folgende Eigenschaft aufweist: mindestens 25% des gesamten Porenvolumens werden durch Poren mit einem Radius zwischen 60 und 400 nm gebildet.

4. Katalysator nach einem der vorstehenden Ansprüche, **dadurch gekennzeichnet, dass** zumindest ein Teil des eingesetzten TiO$_2$ einen Gesamtalkaligehalt von weniger als 1000 ppm aufweist.

5. Katalysator nach einem der vorstehenden Ansprüche, **dadurch gekennzeichnet, dass** die Primärpartikelgröße des eingesetzten TiO$_2$ bei mehr als 250 Angström, insbesondere mehr als 300 Angström, weiter bevorzugt bei mehr als 320 Angström, weiter bevorzugt bei mehr als 340 Angström, weiter bevorzugt bei mehr als 380 Angström liegt.

6. Katalysator nach einem der vorstehenden Ansprüche, **dadurch gekennzeichnet, dass** die BET-Oberfläche des eingesetzten TiO$_2$ zwischen etwa 15 und 60 m$^2$/g, insbesondere etwa 15 und 45 m$^2$/g, besonders bevorzugt 15 und 30 m$^2$/g, liegt.

7. Katalysator nach einem der vorstehenden Ansprüche, **dadurch gekennzeichnet, dass** mindestens etwa 40%, insbesondere mindestens etwa 50% des gesamten Porenvolumens des eingesetzten TiO$_2$ durch Poren mit einem Radius zwischen 60 und 400 nm gebildet werden.

8. Katalysator nach einem der vorstehenden Ansprüche, **dadurch gekennzeichnet, dass** bis zu 70%, insbesondere bis zu 75% des gesamten Porenvolumens des eingesetzten TiO$_2$ durch Poren mit einem Radius zwischen 60 und 400 nm gebildet werden.

9. Katalysator nach einem der vorstehenden Ansprüche, **dadurch gekennzeichnet, dass** etwa 50 bis 70%, insbesondere etwa 50 bis 65% des gesamten Porenvolumens des eingesetzten TiO$_2$ durch Poren mit einem Radius von 60 bis 400 nm gebildet werden.

10. Katalysator nach einem der vorstehenden Ansprüche, **dadurch gekennzeichnet, dass** weniger als 30%, insbesondere weniger als 20% des gesamten Porenvolumens des eingesetzten TiO$_2$ durch Poren mit einem Radius von 3,7 bis 60 nm gebildet werden.

**11.** Katalysator nach einem der vorstehenden Ansprüche, **dadurch gekennzeichnet, dass** etwa 10 bis 30% des gesamten Porenvolumens des eingesetzten $TiO_2$ durch Poren mit einem Radius von 3,7 bis 60 nm gebildet werden.

**12.** Katalysator nach einem der vorstehenden Ansprüche, **dadurch gekennzeichnet, dass** der $D_{90}$-Wert des eingesetzten $TiO_2$ zwischen etwa 1 und 10 $\mu$m, besonders bevorzugt zwischen etwa 2 und 5 $\mu$m liegt.

**13.** Katalysator nach einem der vorstehenden Ansprüche, **dadurch gekennzeichnet, dass** 8 Gew.-% oder mehr der katalytisch aktiven Masse, insbesondere zwischen etwa 8 und 15 Gew.-% an Vanadium, berechnet als Vanadiumpentoxid, vorliegen.

**14.** Katalysator nach einem der vorstehenden Ansprüche, **dadurch gekennzeichnet, dass** der Katalysator mindestens 24 Stunden bei > 390°C, bevorzugt zwischen 24 und 72 Stunden bei > 400°C, in einem $O_2$-haltigen Gas, insbesondere in Luft, calciniert bzw. konditioniert wird.

**15.** Katalysator nach einem der vorstehenden Ansprüche, **dadurch gekennzeichnet, dass** nur eine $TiO_2$-Quelle verwendet wird, wobei das gesamte eingesetzte $TiO_2$ die in einem oder mehreren der vorstehenden Ansprüche definierte BET-Oberfläche bzw. Porenradienverteilung aufweist.

**16.** Verfahren zur Herstellung eines Katalysators gemäß einem der vorstehenden Ansprüche, umfassend die folgenden Schritte:

a. Bereitstellen einer aktiven Masse, enthaltend $TiO_2$, mit einer BET-Oberfläche von mehr als 15 $m^2/g$ und einer Primärkristallitgröße von mehr als 210 Angström und weniger als 900 Angström, bestimmt mittels Pulver-Röntgendiffraktometrie nach der Debye-Scherrer-Formel, wobei der $D_{90}$-Wert des eingesetzten $TiO_2$ zwischen etwa 0,5 und 20 $\mu$m liegt, der $D_{50}$-Wert bei 1,5 $\mu$m oder darunter liegt und weniger als 22% des gesamten Porenvolumens des eingesetzten $TiO_2$ durch Poren mit einem Radius von mehr als 400 nm gebildet werden ,
b. Bereitstellen eines inerten Trägers, insbesondere eines inerten Trägerformkörpers,
c. Aufbringen der katalytisch aktiven Masse auf den inerten Träger insbesondere in einer Wirbelschicht oder einem Fließbett.

**17.** Verwendung eines Titandioxids mit einer BET-Oberfläche von mehr als 15 $m^2/g$ und einer Primärkristallitgröße von mehr als 210 Angström und weniger als 900 Angström, bestimmt mittels Pulver-Röntgendiffraktometrie nach der Debye-Scherrer-Formel, wobei der $D_{90}$-Wert des eingesetzten $TiO_2$ zwischen etwa 0,5 und 20 $\mu$m liegt, der $D_{50}$-Wert bei 1,5 $\mu$m oder darunter liegt und weniger als 22% des gesamten Porenvolumens des eingesetzten $TiO_2$ durch Poren mit einem Radius von mehr als 400 nm gebildet werden, zur Herstellung eines Katalysators, insbesondere zur Gasphasenoxidation von Kohlenwasserstoffen, vorzugsweise zur Gasphasenoxidation von o-Xylol und/oder Naphthalin zu Phthalsäureanhydrid.

**18.** Verwendung gemäß Anspruch 17, **dadurch gekennzeichnet, dass** das Titandioxid eine Schüttdichte von weniger als 1,0 g/ml, vorzugsweise weniger als 0,8 g/ml, insbesondere weniger als 0,6 g/ml aufweist.

**19.** Verwendung gemäß Anspruch 17 oder 18, **dadurch gekennzeichnet, dass** das Titandioxid eine Primärkristallitgröße von mehr als 250 Angström, insbesondere mehr als 300 Angström, weiter bevorzugt bei mehr als 320 Angström, vorzugsweise mehr als etwa 340 Angström, weiter bevorzugt bei mehr als 380 Angström aufweist.

**Claims**

**1.** Catalyst, in particular for preparing phthalic anhydride by gas-phase oxidation of o-xylene and/or naphthalene, comprising an inert support and at least one layer comprising a catalytically active composition containing $TiO_2$ applied thereto, **characterized in that** at least part of the $TiO_2$ used has the following properties: (a) the BET surface area is more than 15 $m^2/g$, (b) the primary crystallite size determined by means of X-ray powder diffraction using the Debye-Scherrer formula is more than 210 Angstrom and less than 900 Angstrom, where the $D_{90}$ of the $TiO_2$ used is in the range from 0.5 to 20 $\mu$m, the $D_{50}$ is 1.5 $\mu$m or below and less than 22% of the total pore volume of the $TiO_2$ used is formed by pores having a radius of more than 400 nm.

**2.** Catalyst according to Claim 1, **characterized in that** the bulk density of the $TiO_2$ used is less than 1.0 g/ml, preferably less than 0.8 g/ml, in particular less than 0.6 g/ml.

3. Catalyst according to either of the preceding claims, **characterized in that** at least part of the $TiO_2$ used has the following property: at least 25% of the total pore volume is formed by pores having a radius in the range from 60 to 400 nm.

4. Catalyst according to any of the preceding claims, **characterized in that** at least part of the $TiO_2$ used has a total alkali metal content of less than 1000 ppm.

5. Catalyst according to any of the preceding claims, **characterized in that** the primary particle size of the $TiO_2$ used is more than 250 Angstrom, in particular more than 300 Angstrom, more preferably more than 320 Angstrom, more preferably more than 340 Angstrom, more preferably more than 380 Angstrom.

6. Catalyst according to any of the preceding claims, **characterized in that** the BET surface area of the $TiO_2$ used is in the range from about 15 to 60 $m^2/g$, in particular from about 15 to 45 $m^2/g$, particularly preferably from 15 to 30 $m^2/g$.

7. Catalyst according to any of the preceding claims, **characterized in that** at least about 40%, in particular at least about 50%, of the total pore volume of the $TiO_2$ used is formed by pores having a radius in the range from 60 to 400 nm.

8. Catalyst according to any of the preceding claims, **characterized in that** up to 70%, in particular up to 75%, of the total pore volume of the $TiO_2$ used is formed by pores having a radius in the range from 60 to 400 nm.

9. Catalyst according to any of the preceding claims, **characterized in that** from about 50 to 70%, in particular from about 50 to 65%, of the total pore volume of the $TiO_2$ used is formed by pores having a radius of from 60 to 400 nm.

10. Catalyst according to any of the preceding claims, **characterized in that** less than 30%, in particular less than 20%, of the total pore volume of the $TiO_2$ used is formed by pores having a radius of from 3.7 to 60 nm.

11. Catalyst according to any of the preceding claims, **characterized in that** from about 10 to 30% of the total pore volume of the $TiO_2$ used is formed by pores having a radius of from 3.7 to 60 nm.

12. Catalyst according to any of the preceding claims, **characterized in that** the $D_{90}$ of the $TiO_2$ used is in the range from about 1 to 10 $\mu$m, particularly preferably from about 2 to 5 $\mu$m.

13. Catalyst according to any of the preceding claims, **characterized in that** 8% by weight or more of the catalytically active composition, in particular from about 8 to 15% by weight of vanadium, calculated as vanadium pentoxide, is present.

14. Catalyst according to any of the preceding claims, **characterized in that** the catalyst is calcined or conditioned in an $O_2$-containing gas, in particular in air, for at least 24 hours at > 390°C, preferably for from 24 to 72 hours at > 400°C.

15. Catalyst according to any of the preceding claims, **characterized in that** only one $TiO_2$ source is used, where the total $TiO_2$ used has the BET surface area or pore radius distribution defined in one or more of the preceding claims.

16. Process for producing a catalyst according to any of the preceding claims, comprising the following steps:

   a. provision of an active composition containing $TiO_2$ having a BET surface area of more than 15 $m^2/g$ and a primary crystallite size of more than 210 Angstrom and less than 900 Angstrom, determined by means of X-ray powder diffraction using the Debye-Scherrer formula, where the $D_{90}$ of the $TiO_2$ used is in the range from about 0.5 to 20 pm, the $D_{50}$ is 1.5 $\mu$m or below and less than 22% of the total pore volume of the $TiO_2$ used is formed by pores having a radius of more than 400 nm,
   b. provision of an inert support, in particular an inert shaped support body,
   c. application of the catalytically active composition to the inert support, in particular in a fluidized bed or a moving bed.

17. Use of a titanium dioxide having a BET surface area of more than 15 $m^2/g$ and a primary crystallite size of more than 210 Angstrom and less than 900 Angstrom, determined by means of X-ray powder diffraction using the Debye-Scherrer formula, where the $D_{90}$ of the $TiO_2$ used is in the range from about 0.5 to 20 $\mu$m, the $D_{50}$ is 1.5 $\mu$m or below and less than 22% of the total pore volume of the $TiO_2$ used is formed by pores having a radius of more than 400 nm, for producing a catalyst, in particular for the gas-phase oxidation of hydrocarbons, preferably for the gas-phase oxidation of o-xylene and/or naphthalene to form phthalic anhydride.

18. Use according to Claim 17, **characterized in that** the titanium dioxide has a bulk density of less than 1.0 g/ml, preferably less than 0.8 g/ml, in particular less than 0.6 g/ml.

19. Use according to Claim 17 or 18, **characterized in that** the titanium dioxide has a primary crystallite size of more than 250 Angstrom, in particular more than 300 Angstrom, more preferably more than 320 Angstrom, preferably more than about 340 Angstrom, more preferably more than 380 Angstrom.

## Revendications

1. Catalyseur, en particulier pour la préparation d'anhydride de l'acide phtalique par oxydation en phase gazeuse d'o-xylène et/ou de naphtalène, présentant un support inerte et au moins une couche, appliquée sur celui-ci, présentant une masse catalytiquement active contenant du $TiO_2$, **caractérisé en ce qu'**au moins une partie du $TiO_2$ utilisé présente les propriétés suivantes : (a) la surface BET est de plus de 15 m$^2$/g, (b) la grosseur des cristaux primaires, déterminée par diffractométrie de rayons X sur poudre selon la formule de Debye-Scherrer, est de plus de 210 Angströms et inférieure à 900 Angströms, la valeur $D_{90}$ du $TiO_2$ utilisé étant située entre 0,5 et 20 $\mu$m, la valeur $D_{50}$ étant de 1,5 $\mu$m ou moins et moins de 22% du volume total de pores du $TiO_2$ utilisé étant formés par des pores présentant un rayon de plus de 400 nm.

2. Catalyseur selon la revendication 1, **caractérisé en ce que** la densité apparente du $TiO_2$ utilisé est de moins de 1,0 g/ml, de préférence de moins de 0,8 g/ml, en particulier de moins de 0,6 g/ml.

3. Catalyseur selon l'une quelconque des revendications précédentes, **caractérisé en ce qu'**au moins une partie du $TiO_2$ utilisé présente la propriété suivante : au moins 25% du volume total de pores sont formés par des pores présentant un rayon entre 60 et 400 nm.

4. Catalyseur selon l'une quelconque des revendications précédentes, **caractérisé en ce qu'**au moins une partie du $TiO_2$ utilisé présente une teneur totale en alcali inférieure à 1000 ppm.

5. Catalyseur selon l'une quelconque des revendications précédentes, **caractérisé en ce que** la grosseur des particules primaires du $TiO_2$ utilisé se situe à plus de 250 Angströms, en particulier à plus de 300 Angströms, plus préférablement à plus de 320 Angströms, encore plus préférablement à plus de 340 Angströms, encore plus préférablement à plus de 380 Angströms.

6. Catalyseur selon l'une quelconque des revendications précédentes, **caractérisé en ce que** la surface BET du $TiO_2$ utilisé se situe entre environ 15 et 60 m$^2$/g, en particulier entre environ 15 et 45 m$^2$/g, de manière particulièrement préférée entre 15 et 30 m$^2$/g.

7. Catalyseur selon l'une quelconque des revendications précédentes, **caractérisé en ce qu'**au moins environ 40%, en particulier au moins environ 50% du volume total de pores du $TiO_2$ utilisé sont formés par des pores présentant un rayon entre 60 et 400 nm.

8. Catalyseur selon l'une quelconque des revendications précédentes, **caractérisé en ce que** jusqu'à 70%, en particulier jusqu'à 75% du volume total de pores du $TiO_2$ utilisé sont formés par des pores présentant un rayon entre 60 et 400 nm.

9. Catalyseur selon l'une quelconque des revendications précédentes, **caractérisé en ce qu'**environ 50% à 70%, en particulier environ 50 à 65% du volume total de pores du $TiO_2$ utilisé sont formés par des pores présentant un rayon de 60 à 400 nm.

10. Catalyseur selon l'une quelconque des revendications précédentes, **caractérisé en ce que** moins de 30%, en particulier moins de 20% du volume total de pores du $TiO_2$ utilisé sont formés par des pores présentant un rayon de 3,7 à 60 nm.

11. Catalyseur selon l'une quelconque des revendications précédentes, **caractérisé en ce qu'**environ 10% à 30% du volume total de pores du $TiO_2$ utilisé sont formés par des pores présentant un rayon de 3,7 à 60 nm.

**12.** Catalyseur selon l'une quelconque des revendications précédentes, **caractérisé en ce que** la valeur $D_{90}$ du $TiO_2$ utilisé se situe entre environ 1 et 10 $\mu$m, de manière particulièrement préférée entre environ 2 et 5 $\mu$m.

**13.** Catalyseur selon l'une quelconque des revendications précédentes, **caractérisé en ce que** 8% en poids ou plus de la masse catalytiquement active, en particulier entre environ 8 et 15% en poids se trouvent sous forme de vanadium, calculé sous forme de pentoxyde de vanadium.

**14.** Catalyseur selon l'une quelconque des revendications précédentes, **caractérisé en ce que** le catalyseur est calciné ou, selon le cas, conditionné pendant au moins 24 heures à > 390°C, de préférence entre 24 et 72 heures à > 400°C, dans un gaz contenant $O_2$, en particulier dans l'air.

**15.** Catalyseur selon l'une quelconque des revendications précédentes, **caractérisé en ce qu'**on n'utilise qu'une source de $TiO_2$, le $TiO_2$ utilisé en totalité présentant la surface BET ou, selon le cas, la distribution des rayons des pores définie dans une ou plusieurs des revendications ci-dessus.

**16.** Procédé pour la préparation d'un catalyseur selon l'une quelconque des revendications ci-dessus, comprenant les étapes suivantes :

> a. préparation d'une masse active, contenant du $TiO_2$ présentant une surface BET de plus de 15 $m^2$/g et une grosseur des cristaux primaires de plus de 210 Angströms et de moins de 900 Angströms, déterminée par diffractométrie de rayons X sur poudre selon la formule de Debye-Scherrer, la valeur $D_{90}$ du $TiO_2$ utilisé étant située entre environ 0,5 et 20 $\mu$m, la valeur $D_{50}$ étant de 1,5 $\mu$m ou moins et moins de 22% du volume total de pores du $TiO_2$ utilisé étant formés par des pores présentant un rayon de plus de 400 nm,
> b. préparation d'un support inerte, en particulier d'un corps façonné support inerte,
> c. application de la masse catalytiquement active sur le support inerte, en particulier dans une couche tourbillonnante ou un lit fluidisé.

**17.** Utilisation d'un dioxyde de titane présentant une surface BET de plus de 15 $m^2$/g et une grosseur des cristaux primaires de plus de 210 Angströms et de moins de 900 Angströms, déterminée par diffractométrie de rayons X sur poudre selon la formule de Debye-Scherrer, la valeur $D_{90}$ du $TiO_2$ utilisé étant située entre environ 0,5 et 20 $\mu$m, la valeur $D_{50}$ étant de 1,5 $\mu$m ou moins et moins de 22% du volume total de pores du $TiO_2$ utilisé étant formés par des pores présentant un rayon de plus de 400 nm, pour la préparation d'un catalyseur, en particulier pour l'oxydation en phase gazeuse d'hydrocarbures, en particulier pour l'oxydation en phase gazeuse d'o-xylène et/ou de naphtalène en anhydride de l'acide phtalique.

**18.** Utilisation selon la revendication 17, **caractérisée en ce que** le dioxyde de titane présente une densité apparente de moins de 1,0 g/ml, de préférence de moins de 0,8 g/ml, en particulier de moins de 0,6 g/ml.

**19.** Utilisation selon la revendication 17 ou 18, **caractérisée en ce que** le dioxyde de titane présente une grosseur des particules primaires de plus de 250 Angströms, en particulier de plus de 300 Angströms, plus préférablement de plus de 320 Angströms, de préférence de plus d'environ 340 Angströms, encore plus préférablement de plus de 380 Angströms.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0522871 A1
- US 20050076811 A1
- EP 1082317 B1
- EP 1084115 B1
- EP 0964744 B1
- DE 2159441 A
- DE 1642938 A1
- DE 1769998 A1
- DE 2106796 A1
- US 2035606 A
- DE 1280756 B
- DE 19828583 A1
- DE 19709589 A1
- EP 744214 A1
- WO 9800778 A1
- EP 714700 A2
- WO 9837967 A1
- WO 9961433 A1
- DE 10040827 A1

**In der Beschreibung aufgeführte Nicht-Patentliteratur**

- Handelsnamen NT22-B20 und NT22-B30. Firma Nano Inc., Ltd, 660-882
- Phtalic Acid and Derivatives. **K. TOWAE ; W. ENKE ; R. JÄCKH ; N. BHARGANA.** Ullmann' s Encyclopedia of Industrial Chemistry. 1992, vol. A. 20, 181
- *J. Am. Chem. Soc,* 1938, vol. 60, 309